# FASCICULE DE BREVET EUROPEEN

(11) **EP 3 094 405 B1**
(45) Date de publication et mention de la délivrance du brevet: **18.12.2019**
(21) Numéro de dépôt: 15705629.2
(22) Date de dépôt: 14.01.2015
(51) Int. Cl.: B01L 3/00, G01N 33/00

(54) **DISPOSITIF MICROFLUIDIQUE POUR L'ANALYSE DE POLLUANTS EN ÉCOULEMENT**
MIKROFLUIDISCHE-VORRICHTUNG ZUR ANALYSE VON SCHADSTOFFEN IM UMLAUF
MICROFLUIDIC DEVICE FOR ANALYSIS OF POLLUTANTS IN CIRCULATION

(30) Priorité: 14.01.2014 FR 1450294; 13.02.2014 FR 1451114
(43) Date de publication de la demande: 23.11.2016
(73) Titulaire: Centre National de la Recherche Scientifique (C.N.R.S.), 75016 Paris (FR); Université de Strasbourg, 67070 Strasbourg (FR)
(72) Inventeur: LE CALVÉ, Stéphane, F-67270 Rohr (FR); ALLOUCH, Alaa El Dine, F-67200 Strasbourg (FR); BERNHARDT, Pierre, F-67190 Heiligenberg (FR); GUGLIELMINO, Maud, F-67310 Wasselonne (FR); SERRA, Christophe, F-67460 Souffelweyersheim (FR)
(74) Mandataire: Priori, Enrico
(86) Numéro de dépôt international: PCT/FR2015/050089
(87) Numéro de publication internationale: WO 2015/107298

(56) Documents cités:
- JP-A- 2005 169 386
- US-A1- 2005 042 615
- US-A1- 2009 012 187
- US-A1- 2009 282 978
- ADAM P. VOLLMER ET AL: "Development of an integrated microfluidic platform for dynamic oxygen sensing and delivery in a flowing medium", LAB ON A CHIP, vol. 5, no. 10, 25 août 2005 (2005-08-25), page 1059, XP055012862, ISSN: 1473-0197, DOI: 10.1039/b508097e

## Description

### DOMAINE DE L'INVENTION

La présente invention concerne un dispositif microfluidique de piégeage et de détection permettant de piéger dans une solution un composé gazeux soluble dans ladite solution puis de le faire réagir avec un agent dérivatif pour former une espèce facilement détectable et quantifiable, par exemple par colorimétrie ou fluorimétrie. L'invention concerne également un procédé de piégeage et de détection d'un composé gazeux mettant en œuvre de tels dispositifs.

### ÉTAT DE LA TECHNIQUE

Le dispositif de la présente invention est particulièrement destiné à l'analyse de polluants gazeux, tels que le formaldéhyde, soluble dans une solution, telle qu'une solution aqueuse.

Le formaldéhyde est présent dans tout notre environnement. Dans l'environnement extérieur, il peut provenir directement des rejets industriels ou automobiles ou encore des feux de forêts ou bien indirectement par oxydation de composés organiques volatils. De par sa grande solubilité dans l'eau, le formaldéhyde se retrouve dans les océans, les eaux de surface ou les eaux de pluie. Le formaldéhyde est également l'un des polluants majeurs de l'air intérieur, il est dégagé entre autres par les peintures, les résines, les bois traités, les papiers traités, les textiles ou encore par les fumées de tabac. Il est généralement présent en environnement intérieur à des concentrations variant typiquement entre 10 et 100 µg/m³ (la concentration en milieu intérieur est typiquement 2 à 15 fois supérieure à la concentration en milieu extérieur). En milieu professionnel, ses concentrations peuvent atteindre plusieurs centaines de µg/m³.

L'impact sanitaire du formaldéhyde est de plus en plus pris en compte par les pouvoirs publics. L'Agence Nationale de Sécurité Sanitaire de l'alimentation, de l'environnement et du travail a fixé un seuil limite de 30 µg/m³ qui ne devra pas être dépassé dans les établissements recevant du public à compter de 2015. Ce seuil sera relevé à 10 µg/m³ à partir de 2022. Le Centre International de Recherche sur le Cancer a également modifié la classification du formaldéhyde en 2006, passant ainsi de la catégorie *cancérogène probable pour l'homme* à la catégorie *cancérogène pour l'homme.*

Une mesure sélective et précise des taux d'émission des matériaux (en µg.m⁻² h⁻¹) et des concentrations dans l'air (µg/m³) apparaît donc comme nécessaire et essentielle.

Plusieurs dispositifs d'analyse de formaldéhyde ont déjà été développés (A. Allouch et al., Transportable, fast and high sensitive near real-time analyzers : Formadehyde detection, Sensors and actuators, B 181 (2013) 551-558). Ces dispositifs comprennent généralement deux étapes successives : une étape de piégeage du formaldéhyde et une étape de détection. La littérature décrit deux méthodes de piégeage du formaldéhyde. Une première méthode repose sur une cellule de piégeage comprenant une surface sensible incluant un adsorbant (par exemple de la cellulose poreuse comprenant un gel de silice) et présentant un changement de couleur de la surface en fonction de l'adsorption du formaldéhyde. Cette méthode permet une grande sélectivité et sensibilité mais est fortement limité par son irréversibilité et son caractère discontinu. Une seconde méthode consiste au transfert du formaldéhyde gazeux dans une solution aqueuse. Un réactif est ensuite mélangé à la solution enrichie en formaldéhyde pour former une espèce facilement détectable. Quelle que soit la méthode de piégeage, l'étape de détection est réalisée ensuite principalement à partir de méthodes chromatographiques, spectroscopiques ou chimiques associées à de la fluorimétrie ou de la colorimétrie.

La demande internationale WO 2010/142908 décrit un dispositif de détermination de la concentration de formaldéhyde gazeux de manière dynamique et en écoulement comprenant (1) une pompe à air pour pomper une quantité prédéterminée de phase gazeuse comprenant le formaldéhyde, (2) des moyens de transfert du formaldéhyde vers une solution aqueuse inerte et (3) des moyens de détermination de la concentration en phase aqueuse du formaldéhyde. Selon un premier mode de réalisation, les moyens de transfert comprennent un capillaire et un tube microporeux. La phase gazeuse comprenant du formaldéhyde est injectée conjointement à une solution aqueuse inerte (en l'espèce de l'eau) dans un tube capillaire. Les gouttelettes qui se forment dans le capillaire sont co-éluées avec le formaldéhyde compris dans la phase gazeuse jusqu'à un tube microporeux qui permet à la phase gazeuse de s'échapper. Selon un second mode de réalisation, les moyens de transfert comprennent un tube microporeux dans lequel circule une solution aqueuse inerte (en l'espèce une solution aqueuse d'acide nitrique ou de l'eau). La phase gazeuse comprenant du formaldéhyde s'écoule alors co-axialement, à l'extérieur et autour du tube microporeux. Le formaldéhyde gazeux est alors transféré vers la solution aqueuse inerte se trouvant à l'intérieur du tube microporeux.

Les moyens de détermination de la concentration en phase aqueuse du formaldéhyde comprennent ensuite des moyens de mélange adaptés pour mélanger du fluoral-p avec d'une part une substance de calibration et d'autre part une quantité prédéterminée de la phase aqueuse enrichie en formaldéhyde au cours de l'étape précédente. Le fluoral-p réagit alors avec le formaldéhyde pour former un composé dérivé (du 3,5-diacetyl-1, 4-dihydrolutidine : DDL) détectable, via une cellule de fluorescence, par spectroscopie de fluorescence.

Des inconvénients limitent encore l'intérêt de ce dispositif, notamment l'obstruction du module de transfert par colmatage du tube microporeux, la consommation importante de réactif de l'ordre de 1 mL/min ou encore la présence d'au moins une substance de calibration dont la concentration en formaldéhyde est connue.

L'un des objectifs de la présente invention réside dans le développement d'un dispositif microfluidique fiable, précis, sensible, autonome et facilement transportable.

Afin d'atteindre ces objectifs, il apparaît important de pouvoir assurer la fiabilité du module de transfert, limiter la consommation de réactif, mais également d'assurer le piégeage et la réaction de dérivation au même emplacement pour abaisser le pas de temps entre deux mesures tout en évitant l'utilisation de substances supplémentaires.

Afin d'atteindre les objectifs de la présente invention, il est également nécessaire d'associer au dispositif microfluidique une cellule de détection offrant une meilleure sensibilité et un temps de renouvellement plus rapide qui permet d'utiliser des débits liquides restreints et donc d'économiser les réactifs.

Le document US 2009/282978 divulgue un dispositif microfluidique de séparation de plusieurs phases dans un écoulement de fluide. Cependant, le dispositif est encombrant et ne permet pas de diminuer le pas de temps entre deux mesures.

Le document US 2009/012187 divulgue un système permettant de produire des émulsions multiples.

### RÉSUMÉ

L'invention concerne donc un dispositif microfluidique d'analyse de composé gazeux, de manière dynamique et en écoulement, ledit dispositif comprenant :
- des moyens de mélange permettant de co-éluer dans un tube capillaire une phase gazeuse comprenant ledit composé gazeux et une solution comprenant un agent dérivatif ;
- des moyens pour faire réagir le composé gazeux en solution avec l'agent dérivatif ;
- des moyens d'élimination de la phase gazeuse ; et
- des moyens de détermination de la concentration en composé gazeux.

Selon la présente invention, les moyens de mélange, permettant de co-éluer dans un tube capillaire une phase gazeuse comprenant ledit composé gazeux et une solution comprenant un agent dérivatif, comprennent une cellule de piégeage comprenant un tube capillaire alimenté en phase gazeuse par un tube capillaire d'alimentation en phase gazeuse et alimenté en solution par un tube d'alimentation en solution.

Selon un mode de réalisation, les moyens pour faire réagir le composé gazeux en solution avec l'agent dérivatif comprennent un moyen de thermorégulation permettant de réguler la température du mélange entre la solution comprenant un agent dérivatif et le composé gazeux initialement en phase gazeuse piégé dans la solution comprenant un agent dérivatif.

Selon un mode de réalisation, les moyens de mélange permettant de co-éluer dans un tube capillaire une phase gazeuse comprenant ledit composé gazeux et une solution comprenant un agent dérivatif, et les moyens pour faire réagir le composé gazeux en solution avec l'agent dérivatif, sont séparés.

Selon un mode de réalisation, les moyens de mélange permettant de co-éluer dans un tube capillaire une phase gazeuse comprenant ledit composé gazeux et une solution comprenant un agent dérivatif, et les moyens pour faire réagir le composé gazeux en solution avec l'agent dérivatif, sont combinés.

Selon la présente invention, les moyens de mélange comprennent des moyens d'obtention d'un flux annulaire.

Selon la présente invention, les moyens d'obtention d'un flux annulaire comprennent un régulateur de débit massique adapté pour les gaz, une pompe à gaz et une pompe péristaltique ou un pousse seringue réglés de façon à ce que le débit gazeux soit 100 à 10000 fois plus important que le débit liquide.

Selon un mode de réalisation, les moyens d'obtention d'un flux annulaire comprennent un régulateur de débit massique adapté pour les gaz, une pompe à gaz et une pompe péristaltique ou un pousse seringue réglés de façon à ce que le débit gazeux soit 850 à 10000 fois plus important que le débit liquide.

Selon un mode de réalisation, le tube capillaire d'alimentation en phase gazeuse et le tube d'alimentation en solution sont perpendiculaires, concentriques ou orientés l'un par rapport à l'autre avec un angle compris entre 0° et 90°, préférentiellement entre 0° et 60°.

Selon la présente invention, lesdits moyens de mélange comprennent un tube capillaire alimenté en son centre par une phase gazeuse comprenant ledit composé gazeux et en périphérie par la solution comprenant un agent dérivatif.

Selon un mode de réalisation, ledit tube capillaire des moyens de mélange est réalisé en matériau hydrophile.

Selon un mode de réalisation, ledit tube capillaire d'alimentation en phase gazeuse est de plus petit diamètre externe que le diamètre interne du tube capillaire, ledit tube capillaire étant inséré partiellement dans le tube capillaire des moyens de mélange.

Selon un mode de réalisation, l'alimentation en solution du tube capillaire des moyens de mélange par le tube d'alimentation en solution est réalisée en amont de l'extrémité distale du tube capillaire d'alimentation en phase gazeuse inséré partiellement dans le premier tube capillaire des moyens de mélange.

Selon la présente invention, le débit gazeux est 100 à 10000 fois plus important que le débit liquide afin d'obtenir un écoulement annulaire.

Selon un mode de réalisation, lesdits moyens de détermination de la concentration en composé gazeux comprennent :
- des moyens de mesure de la concentration du composé dérivé obtenu à partir de la réaction entre le composé gazeux et l'agent dérivatif ; et
- des moyens de calcul de la concentration en composé gazeux à partir de la concentration du composé dérivé obtenue précédemment.

Selon un mode de réalisation, la mesure de la concentration du composé dérivé est réalisée par colorimétrie. Dans ce mode de réalisation, les moyens de mesure de la concentration du composé dérivé comprennent un guide d'onde, une source lumineuse et un détecteur, tel qu'un spectromètre.

Selon un mode de réalisation, la mesure de la concentration du composé dérivé est réalisée par spectroscopie de fluorescence. Dans ce mode de réalisation, les moyens de mesure de la concentration du composé dérivé comprennent une source lumineuse, une cellule de fluorescence comprenant un guide d'onde et un détecteur, tel qu'un photomultiplicateur.

Selon un mode de réalisation, la mesure de la concentration du composé dérivé est réalisée par spectroscopie de fluorescence et colorimétrie.

Un dispositif selon la présente invention peut être mis en œuvre dans le cadre d'un procédé de détermination de la concentration d'un composé gazeux.

Selon un mode de réalisation, le composé gazeux à analyser est le formaldéhyde. Selon un mode de réalisation, la résolution temporelle dudit dispositif est inférieure à 5 min.

Selon un mode de réalisation, la consommation en agent dérivatif liquide est inférieure à 1 mL / min.

### DÉFINITIONS

Dans la présente invention, les termes ci-dessous sont définis de la manière suivante :
- « **Agent dérivatif** » désigne un réactif réagissant avec le composé gazeux à analyser pour former un composé, dit dérivé, facilement détectable et quantifiable.
- « **Co-élution** » fait référence à la co-injection et la coexistence d'une phase gazeuse et d'une phase liquide dans un tube.
- **« Flux annulaire** » fait référence au régime d'écoulement d'un flux diphasique liquide-gaz dans lequel la phase gazeuse circule au cœur de l'écoulement et la phase liquide recouvre complétement la paroi formant ainsi un film annulaire autour de la phase gazeuse. L'obtention d'un flux annulaire est fonction du diamètre interne du tube, de la température et du débit des phases liquides et gazeuses.

On pourra adapter un ou plusieurs de ces paramètres, au vu des enseignements de la présente invention pour obtenir un flux annuaire.
- **« Flux poches-annulaire** » ou « **flux slug-annulaire** » fait référence au régime d'écoulement instable et intermittent d'un flux diphasique liquide-gaz dans lequel la phase gazeuse circule au cœur de l'écoulement et la phase liquide à la périphérie de la phase gazeuse. Ce type d'écoulement comprend en outre partiellement un rétrécissement de l'écoulement gazeux lorsque la phase liquide ne recouvre plus complément la paroi. La phase liquide mouille ainsi partiellement la paroi et forme un film annulaire autour de la phase gazeuse. Ce régime est un régime transitoire entre un flux slug et un flux annulaire. Ainsi, un écoulement annulaire peut comprendre par intermittence un flux slug annulaire. L'obtention d'un flux slug-annulaire est fonction du diamètre interne du tube, de la température et du débit des phases liquides et gazeuses. On pourra adapter l'un ou plusieurs de ces paramètres, au vu des enseignements de la présente invention pour obtenir un flux slug-annuaire.
- « **Flux poches** » ou « **flux slug** » fait référence au régime d'écoulement d'un flux diphasique liquide-gaz comprenant des poches ou bouchons allongés de gaz entourés par un film liquide. L'obtention d'un flux slug est fonction du diamètre interne du tube, de la température et du débit des phases liquides et gazeuses.

On pourra adapter l'un ou plusieurs de ces paramètres, au vu des enseignements de la présente invention pour obtenir un flux slug.
- « **Flux à bulles** » fait référence au régime d'écoulement d'un flux diphasique liquide-gaz comprenant des bulles dispersées dans la phase liquide.
- « **Proximal ou distal** » fait référence au sens d'écoulement du flux dans le dispositif microfluidique ; ainsi proximal fait référence à une position proche de l'entrée de l'écoulement dans un élément du dispositif microfluidique et distal fait référence à une position proche de la sortie de l'écoulement dans ledit élément.
- « **Tube capillaire** » désigne, de façon interchangeable, un tube ou un canal gravé dans un solide, destiné à la circulation de fluides.

### BRÈVE DESCRIPTION DES FIGURES

D'autres particularités et avantages ressortiront clairement de la description qui en est faite ci-après, à titre indicatif et nullement limitatif, en référence aux dessins annexés, dans lesquels :
**Figure 1** est une représentation schématique du dispositif d'analyse selon un mode de réalisation de la présente invention.
**Figure 2** est une représentation schématique du dispositif d'analyse selon un second mode de réalisation de la présente invention.
**Figure 3** est une représentation schématique du dispositif d'analyse selon un troisième mode de réalisation de la présente invention.
**Figure 4** est une représentation schématique d'une cellule de détection selon un mode de réalisation de la présente invention.
**Figure 5** est une vue en coupe d'une cellule de piégeage selon un mode de réalisation de la présente invention.

Les dessins des figures ne sont pas à l'échelle. Il va de soi que la portée de l'invention ne se limite pas aux exemples de réalisation plus spécialement décrits et représentés en référence aux dessins annexés ; elle en embrasse au contraire toutes les variantes.

### RÉFÉRENCES

- **1**: Source de composé gazeux à analyser
- **2**: Solution comprenant un agent dérivatif
- **2'**: Solution inerte sans agent dérivatif
- **2"**: Solution d'agent dérivatif
- **3**: Pompe péristaltique ou pousse seringue
- **4**: Régulateur de débit massique pour gaz
- **5**: Pompe à gaz
- **6**: Cellule de piégeage
- **7**: Moyens d'élimination de la phase gazeuse - Tube microporeux
- **8**: Moyen de thermorégulation - Four
- **9**: Guide d'onde
- **10**: Poubelle
- **11**: Source lumineuse - Lampe Deutérium/Halogène ou lampe xénon pulsée
- **12**: Spectromètre UV-Visible
- **13**: Cellule de détection (fluorescence, colorimétrie ou couplage colorimétrie et fluorescence)
- **14**: Fibre optique
- **15**: Canal, tube ou tube capillaire
- **16**: Source d'excitation pour la fluorescence - LED
- **17**: Photomultiplicateur pour la détection de la fluorescence
- **18**: Tube capillaire hydrophile de la cellule de piégeage
- **19**: Tube capillaire d'alimentation en phase gazeuse de la cellule de piégeage
- **20**: Tube d'alimentation en solution de la cellule de piégeage

### DESCRIPTION DÉTAILLÉE

La présente invention concerne un dispositif microfluidique d'analyse de composé gazeux, de manière dynamique et en écoulement, permettant de piéger dans une solution un composé gazeux soluble et de le faire réagir avec un agent dérivatif pour former une espèce détectable par ledit dispositif.

Ledit dispositif microfluidique d'analyse comprend :
- des moyens de mélange d'une phase gazeuse comprenant ledit composé gazeux et d'un agent dérivatif en solution ;
- des moyens d'élimination de la phase gazeuse ; et
- des moyens de détermination de la concentration en composé gazeux.

Selon la présente invention, le dispositif microfluidique comprend en outre des moyens pour faire réagir le composé gazeux en solution avec l'agent dérivatif.

Selon un mode de réalisation détaillé ci-après, les moyens de mélange comprennent une cellule de piégeage permettant de co-éluer dans un tube capillaire une phase gazeuse comprenant ledit composé gazeux et une solution comprenant un agent dérivatif.

Selon un mode de réalisation détaillé ci-après, les moyens pour faire réagir le composé gazeux en solution avec l'agent dérivatif comprennent des moyens de thermorégulation des fluides et notamment de la solution comprenant l'agent dérivatif.

Dans un mode de réalisation, les moyens de mélange d'une phase gazeuse comprenant ledit composé gazeux et d'un agent dérivatif en solution et les moyens pour faire réagir le composé gazeux en solution avec l'agent dérivatif sont combinés (i.e. mis en œuvre simultanément). Ainsi, dans ce mode de réalisation, la cellule de piégeage est thermo-régulée.

Dans un mode de réalisation alternatif, les moyens de mélange d'une phase gazeuse comprenant ledit composé gazeux et d'un agent dérivatif en solution et les moyens pour faire réagir le composé gazeux en solution avec l'agent dérivatif sont séparés (i.e. mis en œuvre successivement). Ainsi, dans ce mode de réalisation, la cellule de piégeage n'est pas thermo-régulée.

Selon un mode de réalisation dans lequel les moyens de mélange d'une phase gazeuse comprenant ledit composé gazeux et d'un agent dérivatif en solution et les moyens pour faire réagir le composé gazeux en solution avec l'agent dérivatif sont combinés ; les moyens de mélange comprennent des moyens d'obtention d'un flux annulaire.

Selon un mode de réalisation dans lequel les moyens de mélange d'une phase gazeuse comprenant ledit composé gazeux et d'un agent dérivatif en solution et les moyens pour faire réagir le composé gazeux en solution avec l'agent dérivatif sont séparés ; les moyens de mélange comprennent des moyens d'obtention d'un flux annulaire.

Selon les inventeurs, l'obtention d'un flux annulaire et, dans une moindre mesure l'obtention d'un flux poches-annulaire, est particulièrement avantageux car il permet, pour un tube de piégeage donné, d'améliorer le rendement de piégeage et ainsi la sensibilité du dispositif d'analyse. En effet, dans les écoulements à bulles ou à poches, la phase liquide est poussée par les bulles gazeuses et la vitesse linéaire du gaz est sensiblement égale à celle du liquide. Dans le cas d'un écoulement annulaire, la vitesse linéaire du gaz est très supérieure à celle du liquide. Ainsi le temps de séjour du liquide dans la cellule de piégeage, sera très supérieur à celui du gaz.

L'obtention d'un flux annulaire ou slug annulaire est donc important dans le cas où les moyens de mélange et les moyens pour faire réagir le composé gazeux en solution avec l'agent dérivatif sont combinés, ceci afin de garantir un dispositif compact et un temps d'analyse réduit.

Dans le cas où les moyens de mélange d'une phase gazeuse comprenant ledit composé gazeux et d'un agent dérivatif en solution et les moyens pour faire réagir le composé gazeux en solution avec l'agent dérivatif sont séparés ; la taille de la cellule de piégeage peut être augmentée, par exemple en utilisant un tube capillaire en spirale, afin d'augmenter le temps de contact gaz-liquide et de garantir un rendement de piégeage optimal et de fait la même sensibilité analytique quelque soit le type de flux.

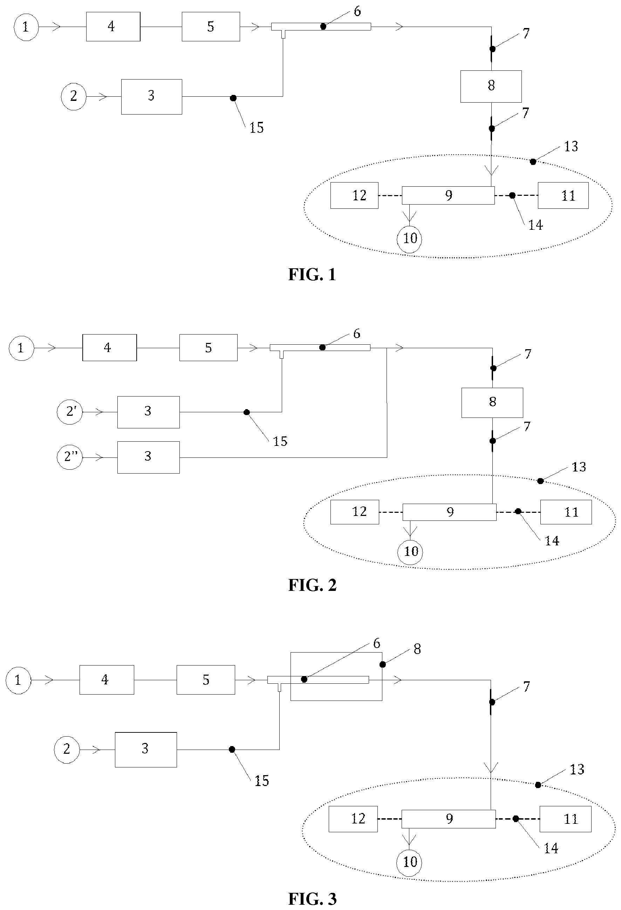

Comme représenté sur les trois modes de réalisation des figures 1, 2 et 3, les moyens de mélange d'une phase gazeuse comprenant un composé gazeux à analyser et un agent dérivatif en solution comprennent au moins un régulateur de débit massique adapté pour les gaz **4**, une pompe à gaz **5** pour réaliser le transport à un débit parfaitement stable d'une phase gazeuse (air ou autre gaz) comprenant le composé gazeux à analyser, une solution, une pompe péristaltique ou un pousse seringue **3** réalisant le transport continu à un débit parfaitement stable de ladite solution et une cellule de piégeage **6.** La pompe à gaz **5** permet d'aspirer une quantité prédéterminée, à l'aide d'un régulateur de débit massique **4**, de phase gazeuse (air ou autre gaz) comprenant le composé gazeux à analyser **1.** Selon un mode de réalisation, le débit de la pompe à gaz **5** est compris entre 0,2 et 100 mL/min, préférentiellement entre 1 et 50 mL/min, encore plus préférentiellement entre 2 et 35 mL/min. Selon un mode de réalisation, des tubes capillaires **15** assurent le transport de la phase gazeuse jusqu'à la cellule de piégeage et ont un diamètre interne compris entre 0,3 et 20 millimètres, de préférence compris entre 0.7 à 8 millimètres. La pompe péristaltique (ou le pousse seringue) **3** permet d'injecter (ou de pousser) une quantité prédéterminée de solution. Selon un mode de réalisation, le débit de la pompe péristaltique **3** est compris entre 0,1 et 100 µL/min, préférentiellement entre 0.1 et 50 µL/min, encore plus préférentiellement entre 0.2 et 35 µL/min. Selon un mode de réalisation, des tubes capillaires **15** assurent le transport de la solution jusqu'à la cellule de piégeage et ont un diamètre interne compris entre 0,2 et 2 millimètres, de préférence compris entre 0,5 à 1 millimètre.

Selon un mode de réalisation préférentiel, représenté sur la figure 1, les moyens de mélange permettent la co-élution, dans une cellule de piégeage **6**, d'une phase gazeuse comprenant ledit composé gazeux **1** et d'une solution **2** comprenant un agent dérivatif.

Selon un mode de réalisation alternatif, représenté sur la figure 2, les moyens de mélange permettent la co-élution, dans une cellule de piégeage **6**, d'une phase gazeuse comprenant ledit composé gazeux à analyser **1** et d'une solution inerte **2'.** L'agent dérivatif **2"** étant alors ajouté en aval de la cellule de piégeage **6.** Dans ce mode de réalisation, le transport de la solution inerte **2'** et de l'agent dérivatif **2"** peut être réalisé à l'aide de deux pompes péristaltiques (ou pousse seringues) **3** ou bien à l'aide d'une pompe péristaltique (ou pousse seringue) **3** à au moins deux canaux. Un premier canal alimente la cellule de piégeage **6** en solution inerte **2'** et un deuxième canal alimente, en aval de la cellule de piégeage, la solution enrichie en composé à analyser, en agent dérivatif **2".**

La cellule de piégeage **6** du composé gazeux **1** est le lieu de mise en contact du composé gazeux à analyser **1** avec une solution **2** ou **2'** afin de transférer le composé gazeux à analyser dans ladite solution. Selon un mode de réalisation de la présente invention, la cellule de piégeage **6** réalise le piégeage du composé gazeux dans un seul tube capillaire cylindrique **18** dans lequel sont co-éluées les phases gazeuse et liquide. Un tel dispositif évite le risque de colmatage des pores des systèmes de piégeage de l'art antérieur à deux tubes, dont un tube microporeux. La cellule de piégeage **6** de la présente invention utilise une phase liquide comprenant directement **2** ou non **2'** l'agent dérivatif. Selon un mode de réalisation, l'écoulement au sein de la cellule de piégeage **6** est diphasique. Selon un mode de réalisation, l'écoulement au sein de la cellule de piégeage **6** comprend au moins une phase liquide (comprenant directement **2** ou non **2'** l'agent dérivatif) et une phase gazeuse (comprenant le composé gazeux à analyser **1**). Selon un mode de réalisation, la cellule de piégeage ne permet pas de générer des émulsions multiples ou émulsions de second ordre.

Selon un mode de réalisation, les moyens de mélange permettent d'imposer l'orientation de l'injection des fluides les uns par rapport aux autres dans la cellule de piégeage **6.** Selon un mode de réalisation, la cellule de piégeage **6** comprend au moins un moyen d'injection de phase gazeuse dans le tube capillaire **18**, tel qu'un tube capillaire d'injection en phase gazeuse **19.** Selon un mode de réalisation, la cellule de piégeage **6** comprend au moins un moyen d'injection de phase liquide dans le tube capillaire **18**, tel qu'un tube d'injection de phase liquide **20.**

Selon un mode de réalisation, l'orientation des flux injectés dans la cellule de piégeage dépend de la disposition des tubes d'alimentations en phase gazeuse et liquide (**19**, **20**). Selon un mode de réalisation, les tubes d'alimentation en phase liquide **20** et en phase gazeuse **19** sont orientés perpendiculairement. Selon un mode de réalisation, les tubes d'alimentation en phase liquide **20** et en phase gazeuse **19** sont orientés parallèlement. Selon un mode de réalisation, les tubes d'alimentation en phase liquide **20** et en phase gazeuse **19** sont agencés de manière concentrique. Selon un mode de réalisation, les tubes d'alimentation en phase liquide **20** et en phase gazeuse **19** sont orientés de manière à former un « Y ». Selon un mode de réalisation, les tubes d'alimentation en phase liquide **20** et en phase gazeuse **19** sont orientés avec un angle compris entre 0° et 90° l'un par rapport à l'autre, préférentiellement entre 0 et 60°. Selon un mode de réalisation, les tubes d'alimentation en phase liquide **20** et en phase gazeuse **19** sont disposés au même niveau dans la cellule de piégeage **6.** Selon un mode de réalisation, les tubes d'alimentation en phase liquide **20** et en phase gazeuse **19** sont décalés (i.e. ne sont pas disposés à la même hauteur dans le tube capillaire **18**) dans la cellule de piégeage **6.** Selon la présente invention, la phase liquide est injectée au niveau de la périphérie du tube capillaire **18** et la phase gazeuse au niveau du centre.

Selon un mode de réalisation, le tube capillaire **18**, préférentiellement la surface interne du tube capillaire cylindrique **18** de la cellule de piégeage, est hydrophile.

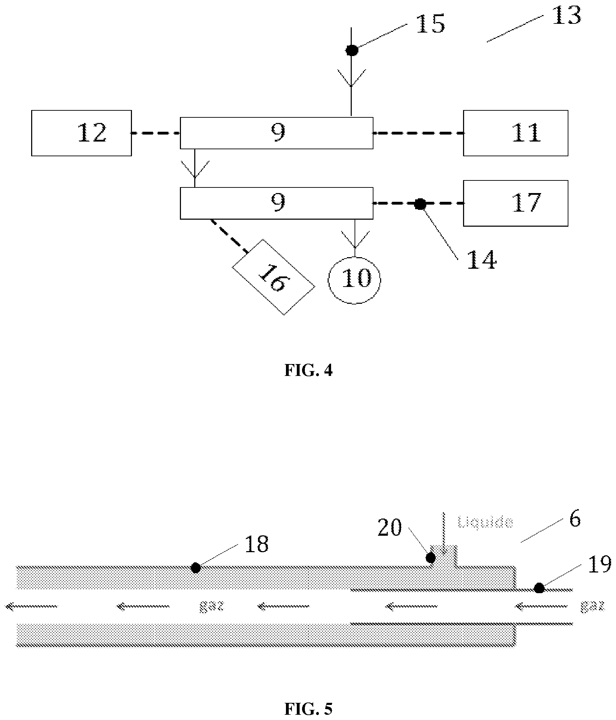

Selon le mode de réalisation, comme représenté sur la figure 5, le composé gazeux est introduit au centre du tube capillaire cylindrique **18** de la cellule de piégeage **6** à l'aide d'un tube capillaire d'alimentation en phase gazeuse **19**, inséré partiellement au centre du tube de piégeage **18.** Une solution comprenant **2** ou non **2'** un agent dérivatif est insérée, à l'aide d'un tube capillaire d'alimentation en phase liquide **20**, au niveau de la paroi du tube **18**, par exemple à l'aide d'un raccord en té, à une hauteur où le tube capillaire **19** est toujours présent. Selon un mode de réalisation, le tube capillaire de la cellule de piégeage **18** est hydrophile, préférentiellement en silice fondue, afin d'obtenir plus facilement un écoulement de type annulaire où la phase gazeuse est située au centre du tube et la phase liquide au niveau des parois du tube. Selon un mode de réalisation, le tube capillaire d'alimentation en phase gazeuse **19** est préférentiellement en silice fondue. Selon un mode de réalisation, le régime d'écoulement annulaire du mélange liquide-gaz est établi dès l'injection des fluides dans la cellule de piégeage **6.** Selon un mode de réalisation, le régime d'écoulement annulaire du mélange liquide-gaz est retardé i.e. n'est pas établi dès l'injection dans la cellule de piégeage **6** mais au cours de la circulation dans la cellule de piégeage **6.**

Selon la présente invention, dans la cellule de piégeage **6**, notamment dans le tube de piégeage **18**, le ratio entre les débits gazeux et liquides est compris entre 100 et 10000, préférentiellement entre 500 et 5000, encore plus préférentiellement 700 et 1500. L'importance du débit gazeux par rapport au débit liquide permet avantageusement de diminuer la consommation de solution.

Selon un mode de réalisation, afin d'obtenir un écoulement annulaire dans la cellule de piégeage, le ratio entre les débits gazeux et liquides est compris entre 850 et 10000, préférentiellement entre 1000 et 5000, encore plus préférentiellement entre 1000 et 3500.

Selon un mode de réalisation, afin d'obtenir un écoulement poches-annulaire dans la cellule de piégeage, le ratio entre les débits gazeux et liquides est compris entre 500 et 1000, plus préférentiellement entre 600 et 850.

Selon un mode de réalisation, afin d'obtenir un écoulement poches dans la cellule de piégeage, le ratio entre les débits gazeux et liquides est compris entre 100 et 600, préférentiellement entre 125 et 550.

Ainsi, par exemple, à température ambiante et pour un diamètre interne du tube de piégeage de 530 µm, on obtient, avec le dispositif selon la présente invention, un flux annulaire pour des ratios de 1000, 1100, 1200, 1400, 1500, 1750, 2000, 2500, 3000, ou 3500 ; un flux poches-annulaire pour des ratios de 600, 750, 800, 850 et un flux poches pour des ratios de 550, 500, 350, 250, 150.

De même, à 65°C et pour un diamètre interne du tube de piégeage de 530 µm, on obtient un flux annulaire pour des ratios de 850, 1000, 1400, 1500, 2000, 3000, 4000, 5000 ; un flux poches-annulaire pour des ratios de 550, 575, 650, 715, 750,800 ; et un flux poches pour des ratios de 150, 200, 250,300, 400, 450, 500.

Selon un mode de réalisation, la cellule de piégeage **6** ne comprend pas de tube microporeux **7.** Selon un mode de réalisation, le diamètre interne du tube capillaire hydrophile de la cellule de piégeage **18** est compris entre 100 et 1000 µm, préférentiellement entre 300 et 700 µm. Selon un mode de réalisation, le diamètre interne du tube capillaire hydrophile de la cellule de piégeage **18** est de 530 µm. Le diamètre externe du tube capillaire d'alimentation en phase gazeuse **19** est inférieur au diamètre interne du tube capillaire **18.** Selon un mode de réalisation, le diamètre externe du tube capillaire d'alimentation en phase gazeuse **19** est compris entre 50 et 850 µm, préférentiellement entre 150 et 500 µm. Selon un mode de réalisation, le diamètre interne du tube capillaire d'alimentation en phase gazeuse **19** est compris entre 20 et 600 µm, préférentiellement entre 50 et 300 µm, encore plus préférentiellement entre 50 et 200 µm. La longueur du tube capillaire hydrophile **18** de la cellule de piégeage est adaptée pour permettre le piégeage total du composé à analyser. Pour un diamètre interne donné, ce paramètre dépend du débit de la phase liquide : à 1 µL/min il faudra une longueur 10 fois plus faible qu'à 10 µL/min pour obtenir le même temps de séjour du liquide dans le tube. Ainsi selon un mode de réalisation, la longueur du tube capillaire **18** peut varier entre 0,5 et 10 m, préférentiellement entre 1 et 5 m pour un débit liquide de 10 µL/min et entre 5 cm et 1 m, préférentiellement entre 10 et 40 cm pour un débit liquide de 1 µL/min. Il est à noter que le diamètre interne du tube capillaire hydrophile **18** influe également sur l'épaisseur du flux annulaire formé et donc sur le temps de séjour du liquide pour un débit liquide donné, et de fait peut modifier la longueur nécessaire du tube capillaire hydrophile **18.** La longueur du tube capillaire d'alimentation en phase gazeuse **19** est adaptée pour permettre un débit de gaz suffisant selon la loi de Poiseuille. Selon un mode de réalisation, la longueur du tube capillaire d'alimentation en phase gazeuse **19** est comprise entre 1 et 50 cm, préférentiellement entre 2 et 20 cm. Selon un mode de réalisation, le tube capillaire **19** est inséré dans le tube capillaire **18** de façon à ce que la paroi externe du tube capillaire **19** ne touche pas la paroi interne du tube capillaire **18.** Selon un mode de réalisation, la distance séparant le tube d'alimentation de la cellule de piégeage en phase liquide **20** de l'extrémité distale du tube capillaire alimentant la cellule de piégeage en phase gazeuse **19** est comprise entre 1 mm et 25 cm, préférentiellement entre 1 mm et 10 cm.

Selon un mode de réalisation, les moyens pour faire réagir le composé gazeux en solution avec l'agent dérivatif comprennent des moyens de thermorégulation des fluides et notamment de la solution. Selon un mode de réalisation, le dispositif microfluidique de la présente invention comprend au moins un moyen de thermorégulation **8.** L'étape de thermorégulation permettant d'accélérer la cinétique de la réaction entre le composé à analyser et l'agent dérivatif est réalisée à l'aide d'un moyen de thermorégulation **8.** Selon un mode de réalisation, le moyen de thermorégulation **8** est situé en aval de la cellule de piégeage **6.** Selon un mode de réalisation, le moyen de thermorégulation **8** est situé au niveau de la cellule de piégeage **6.** Selon un mode de réalisation, ledit moyen, est un four, une résistance chauffante ou tout autre moyen à la portée de l'homme du métier. Selon un mode de réalisation, la température du moyen de thermorégulation **8** est comprise entre 20 et 150°C, préférentiellement entre 40 et 130°C, encore plus préférentiellement entre 50 et 80°C.

Selon un mode de réalisation, comme représenté sur les figures 1 et 2, le flux gazeux est évacué à la sortie de la cellule de piégeage **6** à l'aide de moyens d'élimination de la phase gazeuse **7.** Le tube capillaire **15** comprenant la solution d'agent dérivatif et le composé à analyser piégé dans ladite solution est alors disposé dans un moyen de thermorégulation permettant de régler la température à une valeur favorisant la réaction entre l'agent dérivatif et le composé à analyser. Dans l'exemple particulier où le composé à analyser est le formaldéhyde et le réactif utilisé est le fluoral-p, le moyen de thermorégulation **8**, par exemple un four peut-être à une température comprise entre 20 et 100 °C, préférentiellement entre 50 et 80°C, encore plus préférentiellement 65 °C. L'efficacité de la réaction dépend du temps de séjour du mélange liquide dans le moyen de thermorégulation **8**, qui est fonction à la fois du débit et du volume du capillaire qui dépend quant à lui de la longueur du capillaire et de son diamètre interne. Le capillaire **15**, disposé dans le moyen de thermorégulation **8** peut présenter une longueur comprise entre 0,02 et 10 m, préférentiellement entre 0,05 et 5 m, encore plus préférentiellement entre 0,10 et 2 m. Dans un mode de réalisation, le temps de séjour de la solution dans le moyen de thermorégulation **8** est supérieur à 30 secondes, préférentiellement supérieur à 60 secondes ; encore plus préférentiellement compris entre 60 secondes et 5 minutes; en tout état de cause, le temps de séjour de la solution dans le moyen de thermorégulation **8** est inférieur à 10 minutes, préférentiellement inférieur à 5 minutes. Dans l'exemple particulier où le composé à analyser est le formaldéhyde et le réactif utilisé est le fluoral-p, le temps de séjour de la solution dans le moyen de thermorégulation **8** est préférentiellement supérieur à 2 minutes, encore plus préférentiellement supérieur à 3 minutes ; en tout état de cause, le temps de séjour de la solution dans le moyen de thermorégulation **8** est inférieur à 5 minutes.

Selon un mode de réalisation, représenté sur la figure 3, le moyen de thermorégulation **8** est situé au niveau de la cellule de piégeage **6.** Ce mode de piégeage permet la réalisation du piégeage et de la réaction entre le composé à analyser et l'agent dérivatif au cours de la même étape. Ainsi, le temps de réponse de la mesure entre le moment où la molécule de formaldéhyde gazeux est piégée et le moment où elle est détectée est sensiblement réduit. Le tube capillaire **18** de la cellule de piégeage est disposé dans un moyen de thermorégulation **8** permettant de régler la température à une valeur favorisant la réaction entre l'agent dérivatif et le composé à analyser. Un tel dispositif de piégeage chauffé, à l'aide des moyens de thermorégulation **8**, à une température supérieure à la température ambiante évite avantageusement la condensation de vapeur d'eau autour du tube microporeux des dispositifs de piégeage de l'art antérieur. En effet l'air injecté dans le dispositif de piégeage peut présenter une hygrométrie élevée et un changement de température entre l'air ambiant et le dispositif de piégeage peut entraîner la formation de condensation gênant le piégeage du composé gazeux à analyser à travers le tube microporeux et faussant l'analyse. La présence d'un dispositif de piégeage chauffé comme dans le présent dispositif microfluidique permet de prévenir ce problème. Dans un mode de réalisation où le moyen de thermorégulation **8** est situé au niveau de la cellule de piégeage **6**, les tubes capillaires **15** permettant l'aspiration et le transport de la phase gazeuse jusqu'à la cellule de piégeage **6** sont également chauffés à l'aide d'un moyen de thermorégulation additionnel, tel qu'un four, une résistance chauffante ou tout autre moyen à la portée de l'homme du métier.

Dans l'exemple particulier où le composé à analyser est le formaldéhyde et le réactif utilisé est le fluoral-p, le moyen de thermorégulation **8**, par exemple un four, peut-être à une température comprise entre 20 et 100 °C, préférentiellement entre 50 et 80°C, encore plus préférentiellement 65 °C. Le flux gazeux est alors évacué à la sortie de la cellule de piégeage **6** et du moyen de thermorégulation **8** à l'aide de moyens d'élimination de la phase gazeuse **7.** Dans un mode de réalisation, le temps de séjour de la solution dans le moyen de thermorégulation **8** est supérieur à 30 secondes, préférentiellement supérieur à 60 secondes, encore plus préférentiellement compris en 60 et 5 minutes ;en tout état de cause, le temps de séjour de la solution dans le moyen de thermorégulation **8** est inférieur à 10 minutes, préférentiellement inférieur à 5 minutes. Dans l'exemple particulier où le composé à analyser est le formaldéhyde et le réactif utilisé est le fluoral-p, le temps de séjour de la solution dans le moyen de thermorégulation **8** est préférentiellement supérieur à 2 minutes, encore plus préférentiellement supérieur à 3 minutes ; en tout état de cause, le temps de séjour de la solution dans le moyen de thermorégulation **8** est inférieur à 5 minutes. Ce mode de réalisation est rendu possible grâce à l'écoulement annulaire dans la cellule de piégeage **6** qui permet d'avoir un débit liquide plus faible, et donc un temps de séjour du liquide suffisant pour que la réaction entre le composé à analyser et l'agent dérivatif soit complète, tout en limitant la longueur du tube capillaire **18.**

Selon un mode de réalisation, un moyen de thermorégulation supplémentaire peut être ajouté entre le moyen d'élimination de la phase gazeuse et les moyens de détermination de la concentration en composé gazeux ou entre le moyen de thermorégulation **8** et les moyens de détermination de la concentration en composé gazeux. Ce moyen de thermorégulation supplémentaire est préférentiellement un moyen de refroidissement afin que la solution en écoulement ne soit pas à une température trop importante, préférentiellement à une température comprise entre 10 et 30°C, lors du passage par les moyens de détermination de la concentration en composé gazeux (i.e. lors du passage par la cellule de détection).

Le dispositif selon la présente invention comprend des moyens **7** pour éliminer la phase gazeuse. Ainsi la phase gazeuse comprenant initialement le composé gazeux à analyser et/ou les bulles d'air ou de gaz apparues dans le moyen de thermorégulation et/ou les bulles d'air ou de gaz apparues lors de la réaction entre le composé à analyser et l'agent dérivatif ne perturbent pas, notamment en n'augmentant pas le bruit, les moyens de détermination de la concentration en composé gazeux. La sensibilité et la précision de la mesure sont ainsi améliorées. Selon un mode de réalisation, lesdits moyens **7** sont un ou des tubes microporeux qui laisse(nt) passer les gaz mais pas les liquides. Selon un mode de réalisation, lesdits moyens **7** sont réalisés en toute matière à la portée de l'homme du métier qui soit inerte et poreuse, comme par exemple le téflon microporeux. Dans un mode de réalisation, le tube microporeux est un tube en téflon microporeux. Dans un mode de réalisation, le tube microporeux mesure de 2 à 10 cm de long et de 0,1 à 1,5 mm de diamètre interne.

Selon un mode de réalisation, les moyens **7** pour éliminer la phase gazeuse sont situés entre la cellule de piégeage **6** et le moyen de thermorégulation **8** et/ou entre le moyen de thermorégulation **8** et les moyens de détermination de la concentration en composé gazeux.

Selon un mode de réalisation, les moyens de détermination de la concentration en composé gazeux comprennent :
- des moyens de mesure de la concentration du composé dérivé obtenu à partir de la réaction entre le composé à analyser et l'agent dérivatif ; et
- des moyens de calcul de la concentration en composé gazeux à partir de la concentration du composé dérivé obtenue précédemment.

Les moyens de mesure de la concentration en composé dérivé peuvent comprendre tous moyens de mesure connus de l'homme du métier tel que la fluorimétrie, la colorimétrie, la spectrométrie de masse, etc.

Selon un mode de réalisation, comme représenté sur les figures 1, 2 et 3, les moyens de mesure de la concentration du composé dérivé comprennent une cellule de détection **13** par colorimétrie. Dans ce mode de réalisation, ladite cellule comprend :
- une source lumineuse **11**, préférentiellement une lampe Deutérium/Halogène ou une lampe xénon puisée ;
- un guide d'onde **9**, préférentiellement un guide d'onde à cœur liquide, encore plus préférentiellement un guide d'onde à cœur liquide en Téflon AF2400 ; et
- un spectromètre **12**, de préférence un spectromètre UV-Visible de résolution spectrale de 0,05 à 15 nm, encore plus préférentiellement un spectromètre UV-Visible de résolution spectrale de 1 à 12 nm.

Dans ce mode de réalisation, une première fibre optique **14** relie la source lumineuse au guide d'onde et une seconde fibre optique **14** relie le guide d'onde au spectromètre UV-Visible, afin de collecter l'intensité lumineuse et de limiter tout dérèglement concernant par exemple l'alignement des faisceaux. Selon un mode de réalisation, le diamètre interne du guide d'onde est compris entre 100 µm et 2 mm, préférentiellement entre 200 et 1000 µm. Selon un mode de réalisation, la longueur du guide d'onde est comprise entre 1 et 100 cm, préférentiellement entre 5 et 90 cm, encore plus préférentiellement entre 5 et 40 cm. Préférentiellement le temps de séjour dans le guide d'onde est inférieur à 20 minutes, préférentiellement inférieur 5 minutes, encore plus préférentiellement inférieur à 1,5 minutes afin d'éviter la photolyse du composé dérivé. Pour ce faire on peut modifier le diamètre du guide d'onde et le débit liquide qui, dans le cas du couplage avec la cellule de piégeage microfluidique **6**, est préférentiellement compris entre 0,1 et 100 µL/min, plus préférentiellement entre 1 et 50 µL/min, encore plus préférentiellement entre 2 et 30 µL/min. Dans le cas d'une utilisation de la cellule colorimétrique **9** sans couplage avec la cellule de piégeage microfluidique **6**, le débit peut être augmenté jusqu'à 5 mL/min, préférentiellement jusqu'à 2 mL/min.

Selon un mode de réalisation, la cellule de détection par colorimétrie peut posséder des connectiques SMA (SubMiniature version A) ou tout autre connectique pour fibre optique à la portée de l'homme du métier, permettant de relier d'une part le guide d'onde à une fibre optique reliée à un spectromètre UV-Visible, et d'autre part le guide d'onde à une fibre optique reliée à une source lumineuse. Selon un mode de réalisation alternatif, la source lumineuse et/ou le spectromètre UV-Visible sont accolés au guide d'onde, ce qui permet de s'affranchir de fibres optiques. Dans ce mode de réalisation, un dispositif d'évacuation de la chaleur de la source lumineuse peut être ajouté.

La loi de Beer-Lambert permet de relier l'absorbance et la concentration en composé dérivé piégé en solution. Connaissant la stœchiométrie de la réaction entre le composé à analyser et l'agent dérivatif et le rendement de piégeage du composé gazeux en solution (supposé égal à 100 %), on peut connaître la concentration en composé gazeux à analyser. Dans le cas où le composé à analyser est le formaldéhyde et l'agent dérivatif est le fluoral p, le composé dérivé est la DDL. Comme 1 mole de formaldéhyde réagit avec 2 moles de fluoral-p en excès pour former 1 mole de DDL, on peut calculer le nombre de mole de formaldéhyde expérimentalement piégée en solution à partir de l'absorbance mesurée.

Si le piégeage du formaldéhyde gazeux est considéré comme total, le formaldéhyde gazeux est quantitativement transféré en solution aqueuse, ce qui nous permet de déterminer le nombre de moles de formaldéhyde théoriquement piégé en solution. Le rendement de piégeage est alors le rapport entre le nombre de mole de formaldéhyde expérimentalement piégé et le nombre de mole de formaldéhyde théorique avec un piégeage de 100%. Le rendement de piégeage du dispositif microfluidique selon la présente invention, obtenu pour les concentrations de formaldéhyde gazeux variant entre 20 et 160 µg/m³a été trouvé égal à 88 +/- 12% quelques soient les concentrations.

Les conditions de cet essai ont été les suivantes : débit liquide de 10 µL/min, débit gazeux de 10 mL/min, longueur du tube de piégeage de 2.5 m, diamètre interne du tube de piégeage de 430 µm, température du moyen de thermorégulation de 65°C et temps de séjour du liquide de 3.3 min. Dans cet essai les moyens de mélange de la phase gazeuse comprenant ledit composé gazeux et de l'agent dérivatif en solution et les moyens pour faire réagir le composé gazeux en solution avec l'agent dérivatif étaient combinés (le piégeage est effectué à 65°C).

D'autres essais dans lesquels les moyens de mélange de la phase gazeuse comprenant ledit composé gazeux et de l'agent dérivatif en solution et les moyens pour faire réagir le composé gazeux en solution avec l'agent dérivatif sont combinés ont été réalisés. Les rendements de piégeage du dispositif microfluidique pour des diamètres internes de la cellule de piégeage de 530 µm, 430 µm et 320 µm sont respectivement de 100 +/-16%, 107+/- 18% et 118 +/- 22%. Dans un autre essai, dans lequel les moyens de mélange de la phase gazeuse comprenant ledit composé gazeux et de l'agent dérivatif en solution et les moyens pour faire réagir le composé gazeux en solution avec l'agent dérivatif sont séparés (le piégeage est effectué à température ambiante), le rendement de piégeage du dispositif a été trouvé égal à 90 +/- 2%. Dans cet essai, le débit liquide était de de 50 µL/min, le débit gazeux de 50 mL/min, la longueur du tube de piégeage de 2 m, le diamètre interne du tube de piégeage de 680 µm, la température du moyen de thermorégulation de 65°C et le temps de séjour du liquide dans le four de 4 min.

Selon un mode de réalisation (non représenté), les moyens de mesure de la concentration du composé dérivé comprennent une cellule de détection par fluorimétrie. Dans ce mode de réalisation, ladite cellule comprend :
- au moins une source d'excitation excitant la fluorescence du composé dérivé, préférentiellement au moins une diode électroluminescente (LED) ;
- un guide d'onde, préférentiellement un guide d'onde à cœur liquide, encore plus préférentiellement un guide d'onde à cœur liquide en Téflon AF2400 ; et
- un photomultiplicateur recueillant la fluorescence du composé dérivé.

Dans ce mode de réalisation, une première fibre optique relie la source d'excitation au guide d'onde et une seconde fibre optique relie le guide d'onde au photomultiplicateur, afin de collecter l'intensité lumineuse et de limiter tout dérèglement concernant par exemple l'alignement des faisceaux. Dans le cas où le composé dérivé est la DDL, la source lumineuse peut être une LED émettant à 415 +/- 20 nm excitant la fluorescence de la DDL. Selon un mode de réalisation, un filtre centré sur la longueur d'onde de la fluorescence à mesurer est disposé devant le photomultiplicateur pour éliminer les fluorescences parasites et/ou la lumière parasite émise par la source d'excitation et ainsi recueillir la fluorescence émise uniquement par le composé dérivé. Selon un mode de réalisation, la source d'excitation est placée de telle sorte que l'angle entre la source d'excitation et le guide d'onde soit compris entre 90° et 180°, avec un éclairage en sens opposé à celui de l'écoulement de la solution, plus préférentiellement entre 100 et 160°, encore plus préférentiellement 120°. Selon un mode de réalisation, le diamètre interne du guide d'onde est compris entre 100 µm et 2 mm, préférentiellement entre 300 µm et 1 mm. Selon un mode de réalisation, la longueur du guide d'onde est comprise entre 1 et 20 cm, préférentiellement entre 2 et 10 cm, encore plus préférentiellement entre 3 et 5 cm.

Le guide d'onde transmet la lumière d'excitation et la lumière issue de la fluorescence des molécules. Les molécules du composé dérivé directement éclairées, mais également celles situées en amont de l'éclairage, sont excitées puisque le guide d'onde permet de propager la lumière d'excitation. La lumière de fluorescence, qui est anisotrope dans un système de fluorescence classique, est "piégée" dans le guide d'onde de la présente invention, se propageant ainsi dans les deux sens du guide d'onde dans des proportions probablement similaires. Ainsi, une grande partie de la fluorescence, de l'ordre de 50%, est recueillie sur le détecteur qui est préférentiellement placé dans l'axe du guide d'onde en aval de la source lumineuse afin de limiter le nombre de photons émis par la source qui atteignent le détecteur. La sensibilité des moyens de mesure par fluorimétrie est ainsi grandement améliorée. Selon un mode de réalisation, le volume de la cellule de fluorescence microfluidique est faible et compris entre 0,1 et 100 µL, préférentiellement entre 0,15 µL et 35 µL. Dans une cellule de détection classique en quartz de forme parallélépipédique le renouvellement de la cellule par la solution en écoulement est significativement plus long et le volume est plus important pour une sensibilité identique. Selon un mode de réalisation alternatif, la cellule de détection par fluorimétrie peut comprendre à la place du guide d'onde tout autre type de cellule de fluorescence à la portée de l'homme du métier. Selon un mode de réalisation le diamètre interne du guide d'onde est compris entre 0,05 mm et 5 mm, préférentiellement entre 0,1 mm et 2 mm. Selon un mode de réalisation où la cellule de fluorescence microfluidique **9** est couplée avec la cellule de piégeage microfluidique **6,** le débit liquide est compris entre 0,1 et 100 µL/min, préférentiellement entre 1 et 50 µL/min, encore plus préférentiellement entre 2 et 30 µL/min. Dans le cas d'une utilisation de la cellule de fluorescence sans couplage avec la cellule de piégeage microfluidique **6**, le débit peut être augmenté jusqu'à 5 mL/min, préférentiellement jusqu'à 2 mL/min.

Selon un mode de réalisation, la cellule de détection par fluorimétrie peut posséder des connectiques SMA (SubMiniature version A) ou tout autre connectique pour fibre optique à la portée de l'homme du métier, permettant de relier d'une part le guide d'onde à une fibre optique reliée à un photomultiplicateur, et d'autre part le guide d'onde à une fibre optique reliée à une source d'excitation. Selon un mode de réalisation alternatif, la source d'excitation et/ou le photomultiplicateur sont accolés au guide d'onde, ce qui permet de s'affranchir de fibres optiques. Dans ce mode de réalisation, un dispositif d'évacuation de la chaleur de la source d'excitation peut être ajouté. Selon un mode de réalisation, la cellule de détection comprend au moins une source d'excitation, de préférence 2, 3 ou 4 sources d'excitation afin d'augmenter la lumière d'excitation et donc la fluorescence émise. Selon un mode de réalisation, la paroi du guide d'onde est hydrophobe afin d'éviter les phénomènes d'adsorption du composé dérivé.

Selon un mode de réalisation, les moyens de mesure de la concentration du composé dérivé comprennent une cellule de détection par colorimétrie couplée à une cellule de détection par fluorimétrie. Dans ce mode de réalisation, représenté sur la figure 4, le tube capillaire **15** comprenant le composé dérivé passe à travers un premier guide d'onde **9**, illuminé par une source lumineuse **11** et on détermine alors la concentration absolue (en supposant que le rendement de piégeage est de 100%) en composé dérivé à l'aide du spectromètre **12.** La colorimétrie n'étant pas destructrice, on peut ensuite faire passer la solution dans un deuxième guide d'onde **9**, illuminé par une source d'excitation **16**, on recueille alors la fluorescence des molécules du composé dérivé excité par la source **16** à l'aide d'un photomultiplicateur **17.** La solution est ensuite collectée dans un flacon poubelle **10.**

Dans ce mode de réalisation, le dispositif de la présente invention ne nécessite pas de substance de calibration car la calibration du moyen de mesure par fluorimétrie est réalisée à partir du moyen de mesure par colorimétrie. En effet la colorimétrie est indépendante de l'intensité de la source lumineuse qui va baisser avec le vieillissement du matériel, contrairement à la fluorimétrie qui nécessite une calibration quotidienne. Afin de ne pas nécessiter de substances de calibration, on couple au sein du présent dispositif microfluidique un système de colorimétrie avec un système de fluorimétrie. La fluorimétrie présente néanmoins un intérêt car elle permet la détermination de concentrations plus faibles. Ainsi la cellule de détection par colorimétrie permet de mesurer des concentrations faibles à élevées, typiquement 1 à 2000 µg/m³ avec une limite de détection de 1 µg/m³et la cellule de détection par fluorimétrie permet de mesurer des concentrations très faibles à moyennes, typiquement 0,1 à 500 µg/m³.avec une limite de détection de 0,3 µg/m³. Dans ce mode de réalisation, une première fibre optique **14** relie la source lumineuse **11** au premier guide d'onde **9**, une seconde fibre optique **14** relie le premier guide d'onde **9** au spectromètre **12**, une troisième fibre optique **14** relie la source d'excitation **16** au deuxième guide d'onde **9** et une quatrième fibre optique **14** relie le deuxième guide d'onde **9** au photomultiplicateur **17.** Selon un mode de réalisation alternatif, la ou lesdites fibres optiques **14** peuvent être omises et la source lumineuse **11** et/ou, le spectromètre **12** et/ou la source d'excitation **16** et/ou le photomultiplicateur **17** peuvent être accolés aux guides d'ondes **9.**

Selon un mode de réalisation, les moyens de calculs de la concentration en composé gazeux comprennent un appareil électronique ou informatique, tel qu'un microcontrôleur ou un ordinateur, relié au détecteur, à savoir le spectromètre ou le photomultiplicateur, recevant ainsi le signal brut et calculant la concentration du composé gazeux à analyser dans la phase gazeuse. Selon un mode de réalisation, une interface informatique pilote l'ensemble du dispositif microfluidique.

Le présent document divulgue également un procédé de détermination de la concentration d'un composé gazeux mettant en œuvre le dispositif de la présente invention.

Le composé gazeux analysé par le dispositif microfluidique selon la présente invention est préférentiellement un polluant gazeux soluble, encore plus préférentiellement le formaldéhyde.

Selon un mode de réalisation, le dispositif microfluidique selon la présente invention est utilisé pour déterminer la concentration dans une phase gazeuse de composés gazeux présentant une constante de Henry (H) relativement élevée, c'est-à-dire comprise entre 0,005 M/Pa (20 M/atm) et 2,96 M/Pa (3*10⁵ M/atm). On peut citer à titre d'exemple le formaldéhyde (H = 0,03 M/Pa ou 3100 M/atm), l'hydroperoxyde de méthyle et les composés de la même famille (H = 0,003 M/Pa ou 310 M/atm), le peroxyde d'hydrogène (H = 1,09 M/Pa ou 1,1*10⁵ M/atm), le glyoxal (H = 2,96 M/Pa ou 3,0*10⁵ M/atm), le méthyl glyoxal (H = 0,32 M/Pa ou 3,2*10⁴ M/atm), les acides carboxyliques (H > 0,001 M/Pa ou 1000 M/atm) ou encore le phénol et ses dérivés tels que les crésols (H > 0,005 M/Pa ou 500 M/atm).

Selon un mode de réalisation, l'agent dérivatif utilisé par le dispositif microfluidique selon la présente invention peut être tout agent dérivatif réagissant de manière spécifique avec le composé gazeux à analyser pour former un composé dérivé détectable par des méthodes de détection classiques (colorimétrie, fluorimétrie, etc.). De préférence, l'agent dérivatif présente à la fois un rendement quantique de fluorescence et une vitesse de réaction avec le composé à analyser élevés. Ainsi il a été montré que le fluoral-p réagit préférentiellement avec le formaldéhyde par rapport aux autres aldéhydes présents dans l'air tels que le glyoxal, l'acétaldéhyde ou le hexanal. A température et concentrations égales, la cinétique de la réaction entre le fluoral-p et le formaldéhyde est 3000 à 10000 fois plus rapide. De plus, il a été également montré que le rendement quantique de composés dérivés obtenus à partir de la réaction entre le fluoral-p et les autres aldéhydes présents dans l'air est sensiblement plus faible que le rendement quantique de la DDL.

Selon un mode de réalisation, la solution comprenant un agent dérivatif **2** ou la solution **2'** dans laquelle est ajouté un agent dérivatif **2"** en aval de la cellule de piégeage **6,** est toute solution permettant de solubiliser l'agent dérivatif et le composé à analyser. Selon un mode de réalisation, ladite solution est préférentiellement :
- une solution aqueuse : eau pure, solution aqueuse d'acide nitrique, etc. ;
- un mélange eau-alcool tel qu'un mélange eau/éthanol avec un ratio 90/10 (v/v) ;
- un alcool, tel que l'éthanol ou l'isopropanol.

De préférence, en l'absence d'agent dérivatif, la solution ne réagit pas directement avec le composé à analyser issu de la phase gazeuse et ce composé est parfaitement soluble dans ladite solution. Selon un mode de réalisation, un catalyseur peut être ajouté à la solution pour accélérer la réaction entre l'agent dérivatif et le composé gazeux à analyser.

Selon un mode de réalisation préférentiel, le dispositif microfluidique selon l'invention est utilisé pour déterminer la concentration du formaldéhyde présent dans une phase gazeuse avec comme agent dérivatif le fluoral-p. Le fluoral-p réagit spécifiquement avec le formaldéhyde pour former le 3, 5-diacetyl-1, 4-dihydrolutidme (ou DDL).

Le dispositif microfluidique selon la présente invention permet une diminution de la consommation de solution **2** ou **2'** et **2"** et permet une augmentation de l'autonomie de l'appareil, notamment grâce à l'utilisation d'un écoulement annulaire dans la cellule de piégeage. Selon un mode de réalisation, la consommation en solution est inférieure à 1 mL/min, de l'ordre de 10 µL/min. Ainsi avec le dispositif décrit dans la présente invention, 10 mL de réactifs suffisent pour réaliser près de 500 analyses. À titre de comparaison, le dispositif décrit dans la demande internationale WO 2010/142908 ne permettait de réaliser qu'une seule analyse avec la même quantité de réactif. L'autonomie et l'encombrement du dispositif sont sensiblement améliorés.

Le dispositif microfluidique selon la présente invention permet une diminution de la résolution temporelle du dispositif, notamment grâce à la réalisation du piégeage et de la réaction de dérivation *in situ.* Selon un mode de réalisation, la résolution temporelle du présent dispositif est inférieure à 5 minutes, préférentiellement inférieure à 2 minutes, encore plus préférentiellement de l'ordre de 1 minute. Selon un mode de réalisation, le délai de réponse initiale du dispositif est inférieur à 20 minutes, préférentiellement inférieur à 10 minutes, encore plus préférentiellement de l'ordre de 5 à 6 minutes.

Selon un mode de réalisation, le dispositif microfluidique est facilement transportable ; il est réalisé avec des moyens peu volumineux et légers et nécessite peu de réactifs **2** ou **2'** et **2".** Selon un mode de réalisation, un microcontrôleur et/ou un dispositif d'affichage sont intégrés au dispositif selon la présente invention afin de garantir un dispositif parfaitement autonome.

Selon un mode de réalisation, la solution **2** ou les solutions **2'** et **2"** sont stockées à l'intérieur du dispositif microfluidique dans un flacon ou une microplaque facilement remplaçable une fois la solution consommée.

Selon un mode de réalisation, l'ensemble des capillaires **15** du dispositif, hormis le tube capillaire **18** du système de piégeage **6** et le(s) tube(s) microporeux **7,** est réalisé en PEEK ou tout autre matériau à la portée de l'homme du métier.

## Revendications

1. Dispositif microfluidique d'analyse de composé gazeux, de manière dynamique et en écoulement, ledit dispositif comprenant :
- des moyens de mélange permettant de co-éluer dans un tube capillaire (18) une phase gazeuse comprenant ledit composé gazeux (1) et une solution comprenant un agent dérivatif (2), les moyens de mélange comprenant, pour réaliser le transport de la phase gazeuse, un régulateur de débit massique adapté pour les gaz (4) ou une pompe à gaz (5) et, pour réaliser le transport de la solution, une pompe péristaltique ou un pousse seringue (3), ces moyens étant réglés de façon à ce que le débit gazeux soit 100 à 10000 fois plus important que le débit liquide ;
- des moyens pour faire réagir le composé gazeux en solution avec l'agent dérivatif (2) ;
- des moyens d'élimination de la phase gazeuse (7) ; et
- des moyens de détermination de la concentration en composé gazeux,
**caractérisé en ce que** lesdits moyens de mélange permettent l'obtention d'un flux annulaire et comprennent une cellule de piégeage (6) comprenant un tube capillaire (18) alimenté en son centre en phase gazeuse comprenant ledit composé gazeux par un tube capillaire d'alimentation en phase gazeuse (19) et en périphérie en solution comprenant ledit agent dérivatif par un tube d'alimentation en solution (20).

2. Le dispositif selon la revendication **1**, dans lequel les moyens pour faire réagir le composé gazeux en solution avec l'agent dérivatif (2) comprennent un moyen de thermorégulation permettant de réguler la température du mélange entre la solution comprenant un agent dérivatif (2) et le composé gazeux (1) initialement en phase gazeuse piégé dans la solution comprenant un agent dérivatif (2).

3. Le dispositif selon l'une quelconque des revendications **1** à **2**, dans lequel les moyens de mélange permettant de co-éluer dans un tube capillaire (18) une phase gazeuse comprenant ledit composé gazeux (1) et une solution comprenant un agent dérivatif (2) et les moyens pour faire réagir le composé gazeux en solution avec l'agent dérivatif (2) sont séparés.

4. Le dispositif selon l'une quelconque des revendications **1** à **2**, dans lequel les moyens de mélange permettant de co-éluer dans un tube capillaire (18) une phase gazeuse comprenant ledit composé gazeux (1) et une solution comprenant un agent dérivatif (2) et les moyens pour faire réagir le composé gazeux en solution avec l'agent dérivatif (2) sont combinés.

5. Le dispositif selon l'une des revendications **1** à **4**, dans lequel les moyens de mélange sont réglés de façon à ce que le débit gazeux soit 850 à 10000 fois plus important que le débit liquide.

6. Le dispositif selon l'une quelconque des revendications **1** à **5**, dans lequel le tube capillaire (19) d'alimentation en phase gazeuse et le tube d'alimentation en solution (20) sont perpendiculaires, concentriques ou orientés l'un par rapport à l'autre avec un angle compris entre 0° et 90°.

7. Le dispositif selon l'une quelconque des revendications **1** à **6**, dans lequel le tube capillaire (19), d'alimentation en phase gazeuse est de plus petit diamètre externe que le diamètre interne du tube capillaire (18) des moyens de mélange, ledit tube capillaire (19) étant inséré partiellement dans le tube capillaire (18) des moyens de mélange.

8. Le dispositif selon la revendication **7**, dans lequel l'alimentation en solution du tube capillaire (18) des moyens de mélange par le tube d'alimentation en solution (20) est réalisée en amont de l'extrémité distale du tube capillaire (19) d'alimentation en phase gazeuse.

9. Le dispositif selon l'une quelconque des revendications **1** à 8, dans lequel les moyens de détermination de la concentration en composé gazeux comprennent :
- des moyens de mesure de la concentration du composé dérivé obtenu à partir de la réaction entre le composé gazeux et l'agent dérivatif ; et
- des moyens de calcul de la concentration en composé gazeux à partir de la concentration du composé dérivé obtenue précédemment.

10. Le dispositif selon la revendication **9**, dans lequel la mesure de la concentration du composé dérivé est réalisée par colorimétrie et/ou par spectroscopie de fluorescence.

11. Le dispositif selon l'une quelconque des revendications **9** ou **10**, dans lequel les moyens de mesure de la concentration du composé dérivé comprennent un guide d'onde (9), une source lumineuse (11) et un détecteur (12).

## Patentansprüche

1. Mikrofluidische Vorrichtung zur dynamischen Analyse einer gasförmigen Verbindung im Umlauf, wobei die Vorrichtung Folgendes beinhaltet:
- Mischmittel, welche es ermöglichen, in einer Kapillarröhre (18) eine Gasphase, welche die gasförmige Verbindung (1) beinhaltet und eine Lösung, welche ein Derivativmittel (2) beinhaltet, gleichzeitig zu eluieren, wobei die Mischmittel zum Transport der Gasphase einen Massendurchflussregler beinhalten, welcher für die Gase (4) geeignet ist oder eine Gaspumpe (5), und, zum Transport der Lösung, eine peristaltische Pumpe oder einen Spritzenkolben (3), wobei diese Mittel so eingestellt sind, dass der Gasdurchfluss 100 bis 10000 Mal höher als der Flüssigkeitsdurchfluss ist;
- Mittel zum Umsetzen der gasförmigen Verbindung in der Lösung mit dem Derivativmittel (2);
- Mittel zum Beseitigen der Gasphase (7); und
- Mittel zum Bestimmen der Konzentration an gasförmiger Verbindung,
**dadurch gekennzeichnet, dass** die Mischmittel es ermöglichen, einen ringförmigen Fluss zu erzielen und eine Fangzelle (6) beinhalten, welche eine Kapillarröhre (18) beinhalten, welche in ihrem Mittelpunkt mit Gasphase gespeist wird, welche die gasförmige Verbindung beinhaltet, durch eine Gasphasen-Kapillarspeiseröhre (19), und an der Peripherie mit Lösung, welche das Derivativmittel beinhaltet, durch eine Lösungs-Speiseröhre (20).

2. Vorrichtung nach Anspruch 1, bei welcher die Mittel zum Umsetzen der gasförmigen Verbindung in Lösung mit dem Derivativmittel (2) ein Wärmeregelungsmittel enthalten, welches es ermöglicht, die Temperatur der Mischung zwischen der ein Derivativmittel (2) enthaltenden Lösung und der ursprünglich in der Gasphase vorliegenden gasförmigen Verbindung (1), welche in der das Derivativmittel (2) enthaltenden Lösung gefangen ist, zu regulieren.

3. Vorrichtung nach einem der Ansprüche 1 bis 2, bei welcher die Mischmittel, welche es ermöglichen, in einer Kapillarröhre (18) eine Gasphase, welche die gasförmige Verbindung (1) beinhaltet und eine Lösung, welche ein Derivativmittel (2) beinhaltet, gleichzeitig zu eluieren, und die Mittel zum Umsetzen der gasförmigen Verbindung in der Lösung mit dem Derivativmittel (2) getrennt sind.

4. Vorrichtung nach einem der Ansprüche 1 bis 2, bei welcher die Mischmittel, welche es ermöglichen, in einer Kapillarröhre (18) eine Gasphase, welche die gasförmige Verbindung (1) beinhaltet und eine Lösung, welche ein Derivativmittel (2) beinhaltet, gleichzeitig zu eluieren, und die Mittel zum Umsetzen der gasförmigen Verbindung in der Lösung mit dem Derivativmittel (2) kombiniert sind.

5. Vorrichtung nach einem der Ansprüche 1 bis 4, bei welcher die Mischmittel so eingestellt sind, dass der Gasdurchfluss 850 bis 10000 Mal höher als der Flüssigkeitsdurchfluss ist.

6. Vorrichtung nach einem der Ansprüche 1 bis 5, bei welcher die Kapillarröhre (19) zur Speisung mit Gasphase und die Kapillarröhre zur Speisung mit Lösung (20) zueinander rechtwinklig, konzentrisch oder mit einem Winkel zwischen 0° und 90° ausgerichtet sind.

7. Vorrichtung nach einem der Ansprüche 1 bis 6, bei welcher die Kapillarröhre (19) zur Speisung mit Gasphase einen kleineren Außendurchmesser aufweist als der Innendurchmesser der Kapillarröhre (18) der Mischmittel, wobei die Kapillarröhre (19) teilweise in die Kapillarröhre (18) der Mischmittel eingesetzt wird.

8. Vorrichtung nach Anspruch 7, bei welcher die Speisung mit Lösung der Kapillarröhre (18) der Mischmittel durch die Lösungs-Speiseröhre (20) im vorgelagerten Bereich des distalen Endes der Kapillarröhre (19) zur Speisung mit Gasphase erfolgt.

9. Vorrichtung nach einem der Ansprüche 1 bis 8, bei welcher die Mittel zum Bestimmen der Konzentration an gasförmiger Verbindung Folgendes beinhalten:
- Mittel zum Messen der Konzentration der anhand der Reaktion zwischen der gasförmigen Verbindung und dem Derivativmittel erzielten Derivatverbindung; und
- Mittel zum Berechnen der Konzentration an gasförmiger Verbindung anhand der zuvor erzielten Konzentration an Derivatverbindung.

10. Vorrichtung nach Anspruch 9, bei welcher die Messung der Konzentration an Derivatverbindung durch Kolorimetrie und/oder durch Fluoreszenzspektroskopie erfolgt.

11. Vorrichtung nach einem der Ansprüche 9 oder 10, bei welcher die Mittel zur Messung der Konzentration an Derivatverbindung einen Hohlwellenleiter (9), eine Lichtquelle (11) und einen Detektor (12) beinhalten.

## Claims

1. Microfluidic device for analysing a gaseous compound, in a dynamic manner and in a flow, the device comprising:
- mixing means which enable co-elution in a capillary tube (18) of a gaseous phase comprising the gaseous compound (1) and a solution comprising a derivative agent (2), the mixing means comprising, in order to carry out the transport of the gaseous phase, a mass flow controller which is adapted for gases (4) or a gas pump (5), and, in order to carry out the transport of the solution, a peristaltic pump or a syringe pump (3), these means being controlled so that the gas flow is from 100 to 10000 times greater than the liquid flow;
- means for reacting the gaseous compound in solution with the derivative agent (2);
- means for eliminating the gaseous phase (7); and
- means for determining the concentration of gaseous compound,
**characterised in that** the mixing means enable an annular flow to be obtained and comprise an entrapment cell (6) comprising a capillary tube (18) which is supplied at the centre thereof with gaseous phase comprising the gaseous compound via a gaseous phase capillary supply tube (19) and at the periphery with a solution comprising the derivative agent via a solution supply tube (20).

2. The device according to claim 1, wherein the means for reacting the gaseous compound in solution with the derivative agent (2) comprise a thermoregulation means which allows control of the temperature of the admixture between the solution comprising a derivative agent (2) and the gaseous compound (1) initially in a gaseous phase trapped in the solution comprising a derivative agent (2).

3. Device according to any one of claims 1 to 2, wherein the mixing means enabling co-elution in a capillary tube (18) of a gaseous phase comprising the gaseous compound (1) and a solution comprising a derivative agent (2) and the means for reacting the gaseous compound in solution with the derivative agent (2) are separated.

4. Device according to any one of claims 1 to 2, wherein the mixing means enabling co-elution in a capillary tube (18) of a gaseous phase comprising the gaseous compound (1) and a solution comprising a derivative agent (2) and the means for reacting the gaseous compound in solution with the derivative agent (2) are combined.

5. Device according to any one of claims 1 to 4, wherein the mixing means are controlled so that the gaseous flow is from 850 to 10000 times greater than the liquid flow.

6. Device according to any one of claims 1 to 5, wherein the gaseous phase capillary supply tube (19) and the solution supply tube (20) are perpendicular, concentric or orientated relative to each other at an angle between 0° and 90°.

7. Device according to any one of claims 1 to 6, wherein the gaseous phase capillary supply tube (19) has a smaller external diameter than the internal diameter of the capillary tube (18) of the mixing means, the capillary tube (19) being inserted partially into the capillary tube (18) of the mixing means.

8. Device according to claim 7, wherein the supply of solution to the capillary tube (18) of the mixing means via the solution supply tube (20) is produced upstream of the distal end of the gaseous phase capillary supply tube (19).

9. Device according to any one of claims 1 to 8, wherein the means for determining the concentration of gaseous compound comprise:
- means for measuring the concentration of the derived compound obtained from the reaction between the gaseous compound and the derivative agent; and
- means for calculating the concentration of gaseous compound from the concentration of the derivative compound obtained previously.

10. Device according to claim 9, wherein the measurement of the concentration of the compound derived is produced by means of colorimetry and/or by means of fluorescence spectroscopy.

11. Device according to any one of claim 9 or claim 10, wherein the means for measuring the concentration of the derived compound comprise a wave guide (9), a light source (11) and a detector (12).
